# EUROPEAN PATENT APPLICATION

(11) **EP 3 539 536 A1**
(43) Date of publication of application: **18.09.2019**
(21) Application number: 18161959.4
(22) Date of filing: 15.03.2018
(51) Int. Cl.: A61K 9/16, A61K 31/404

(54) **A PHARMACEUTICAL COMPOSITION OF SUNITINIB OR ITS SALT THEREOF IN ITS POLYMORPHIC FORM I**

(71) Applicant: MH10 Spolka z ograniczona odpowiedzialnoscia, 00-728 Warsaw (PL)
(72) Inventor: Kurek, Joanna, 02-673 Warsaw (PL); Rapacz, Joanna, 05-400 Otwock (PL); Popio kiewicz, Joanna, 05-800 Pruszków (PL); Urba ska, Agnieszka, 05-110 Jab onna (PL); Sijka, Beata, 02-943 Warszawa (PL); Wi niewska, Anna, 01-873 Warszawa (PL); Osi ski, Andrzej, 00-875 Warszawa (PL); ukaszewicz, Piotr, 01-401 Warszawa (PL); Hejduk, Arkadiusz, 05-462 Wi zowna (PL)
(74) Representative: Kondrat, Mariusz

(57) **Abstract**

The invention relates to a pharmaceutical composition of sunitinib or its salt thereof in its polymorphic form I allowing for preparation of all proportional doses of sunitinib composition for administration to a patient comprising diluent, disintegrant, binder, lubricant wherein the composition comprises a macromolecular polymer - hypromellose (HPMC) in amount of 1-5% by weight of the total weight of the composition.

The invention also relates to a method of producing pharmaceutical composition of sunitinib or its salt.

## Description

### Field of the invention

The present invention refers to a pharmaceutical composition of sunitinib and the salt thereof allowing for preparation of all doses of final composition for administration to a patient as active pharmaceutical ingredient.

The invention relates to a pharmaceutical composition comprising N-[2-(Diethylamino)ethyl]-5-[(Z)-(5-fluoro-1,2-dihydro-2-oxo-3H-indol-3-ylidene)methyl]-2,4-dimethyl-1H-pyrrole-3-carboxamide (2S)-2-hydroxybutanedioic acid (1:1) salt, and the salt thereof in its polymorphic form I and a process of preparing such composition.

### Background of the invention

In WO2004024127 (EP1536783 B2) there is disclosed a pharmaceutical formulation of indolinones, including sunitinib, which is described as readily formed into an oral capsule or tablet.
The polymorphic form I of sunitinib and method of preparing maleate salt is known form the patent application WO2009074862. The crystals including a malic acid salt of N-[2-(diethylamino)ethyl]-5-[(5-fluoro-1,2-dihydro-2-oxo-3H-indol-3-ylidene)methyl]-2,4-dimethyl-1H-pyrrole-3-carboxamide and compositions thereof were disclosed in WO03016305.

Generally, Sunitinib is administered in a dose of 50 mg once daily, which, can be varied according to individual tolerance and safety. In order to obtain sufficiently flexible dosing, individual dosage forms generally contain 12.5, 25 and 50 mg Sunitinib. Capsules comprising the malate salt of Sunitinib are sold under the brand name Sutent® (by Pfizer Pharma).

WO2004024127 discloses agranular composition consisting of 40% w/w of 5-(5-fluoro-2-oxo-1,2-dihydroindol-3-ylidenemethyl)-2,4-dimethyl-1H-pyrrole-3-carboxylic acid (2-diethylamino-ethyl)-amide L-malate; 47.5% w/w of mannitol; 6.0% w/w of croscarmellose sodium; 5.0% w/w of povidone (K-25); and 1.5% w/w of magnesium stearate wherein half the amount of croscarmellose sodium is intragranular, and half the amount of croscarmellose sodium together with the whole amount of magnesium stearate are extragranular.

However, a deeper analysis of the disclosed examples leads to a conclusion that the proposed technology does not allow for production of all applied doses of sunitinib maleate i.e. 50mg, 37.5 mg, 25mg, 12.5mg in the final product and the dose of 12,5 mg of sunitinib maleate is capsuled from a separate formulation having different content of the ingredients.

Therefore, there is still a need in the field for a stable, uniform formulation of sunitinib, which can be readily formed into all dosage forms and which is substantially free of the disadvantages of formulations disclosed in the prior art.

It was found that the above-mentioned problems can be solved by the addition of hypromellose (HPMC) in an amount of 1-5% by weight of the total weight of the composition.

### Description of the invention

The invention provides a pharmaceutical composition of sunitinib maleate, which can be easily formulated into an oral capsule or tablet of all doses administered to a patient, i.e. 50mg, 25mg and 12.5mg. The pharmaceutical composition comprises sunitinib maleate and diluent, disintegrant, binder, lubricant and a macromolecular polymer - hypromellose (HPMC) in amount of 1-5% by weight of the total weight of the composition.

Sunitinib can be synthesized using techniques well known in the art. The synthesis of Sunitinib is disclosed for example in WO0160814.

The ability to readily prepare each of the available compositions, containing sunitinib maleate polymorph I, besides a diluent, disintegrant, binder, lubricant, is provided by the addition of hypromellose (HPMC) in an amount of 1-5% by weight of the total weight of the composition.

A pharmaceutical composition of sunitinib or its salt thereof in its polymorphic form I allowing for preparation of all proportional doses of sunitinib composition for administration to a patient comprising diluent, disintegrant, binder, lubricant wherein the composition comprises a macromolecular polymer - hypromellose (HPMC) in amount of 1-5% by weight of the total weight of the composition.
Preferably, the pharmaceutical composition comprises sunitinib maleate.
Preferably, the pharmaceutical composition comprises the following components:

| | |
|---|---|
| sunitinib malate | 35-45 % w/w, |
| mannitol | 35-45% w/w, |
| croscarmellose sodium | 5-10% w/w, |
| povidone (K-25) | 2-8% w/w, |
| hypromellose | 1-5% w/w, |
| magnesium stearate | 1-2% w/w. |

Preferably, the pharmaceutical composition comprises the following components:

| | |
|---|---|
| sunitinib malate, | 39,8 % w/w |
| mannitol, | 42,7% w/w |
| croscarmellose sodium (8% intragranular), | 8,0% w/w |
| povidone (K-25), | 5,0% w/w |
| hypromellose, | 3,0% w/w |
| magnesium stearate | 1,5% w/w |

Preferably, the pharmaceutical composition, has bulk density in the range from 0.35 to 0.50 g/cm³ and a Hausner ratio of 1.1 to 1.4.
Preferably, the composition is characterized in that the active ingredient is present in the form of particles having a d90 value of less than 150 µm, d50 within the range of 10 to 60 µm, d10 within th range of 0 to 10 µm.

Preferably, the pharmaceutical composition has the form of capsules.

A method for manufacture of pharmaceutical composition according to the invention comprising wet granulation of sunitinib or its salt thereof in its polymorphic form I and diluent, disintegrant, binder, macromolecular polymer, which is hypromellose (HPMC), then the granulate is dried and calibrated and a lubricant is added into granulate, further the granulate is formulated into pharmaceutical form of capsule.

The pharmaceutical composition comprises macromolecular polymer - hypromellose (HPMC), in the amount of 1 - 5% w/w. Hypromellose is a factor controlling the rate of release of sunitinib malate from the pharmaceutical form. Thanks to its use in the composition From one universal composition is possible to apply proportional technology of producing all doses of the final drug product.
The pharmaceutical composition has bulk density in the range from 0.35 to 0.50 g/cm³ and a Hausner ratio of 1.1 to 1.4 measured according to Ph.Eur. 2.9.34. Particle size distribution for tested API is as follows: a d90 value of less than 150 µm, d50 within the range of 10 to 60 µm, d10 within th range of 0 to 10 µm measured on Malvern Laser Diffraction Mastersizer 2000 apparatus.
The pharmaceutical composition has the form of capsules.

The invention is further illustrated by the following examples, which are not constructed as being limiting.
Pharmaceutical composition is obtained by wet granulation process comprising sunitinib malate and following excipients: mannitol, croscarmellose sodium, povidone, hypromelose. The process is conducted in high-shear granulator. The granulate obtained in the granulation process is dried and calibrated. Magnesium stearate is added into granulate as a lubricant. Subsequently, the granulate is encapsulated in gelatine capsules. Capsules are packed into blisters.

### Example 1 In a preferred embodiment, the composition comprises:

**Table 1**

| **Ingredient Name** | **Concentration in granulation (% w/w)** | **Amount in 50 mg Capsule [mg]** | **Amount in 25 mg Capsule [mg]** | **Amount in 12,5 mg Capsule [mg]** |
|---|---|---|---|---|
| Sunitinib Malate | 39,8 | 66,80 | 33,4 | 16,70 |
| Mannitol | 42,7 | 71,72 | 35,86 | 17,93 |
| Croscarmellose Sodium | 8,0 | 13,52 | 6,76 | 3,38 |
| Povidone (K-25) | 5,0 | 8,40 | 4,20 | 2,10 |
| Hypromellose | 3,0 | 5,04 | 2,52 | 1,26 |
| Magnesium stearate | 1,5 | 2,52 | 1,26 | 0,63 |
| Capsule | - | Gelatin | Gelatin | Gelatin |

### Example 2 In a preferred embodiment, the composition comprises:

**Table 2**

| **Ingredient Name** | **Concentration in granulation (% w/w)** | **Amount in 50 mg Capsule [mg]** | **Amount in 25 mg Capsule [mg]** | **Amount in 12,5 mg Capsule [mg]** |
|---|---|---|---|---|
| Sunitinib Malate | 39,8 | 66,80 | 33,4 | 16,70 |
| Mannitol | 44,7 | 75,16 | 37,58 | 18,79 |
| Croscarmellose Sodium | 6,0 | 10,08 | 5,04 | 2,52 |
| Povidone (K-25) | 5,0 | 8,40 | 4,20 | 2,10 |
| Hypromellose | 3,0 | 5,04 | 2,52 | 1,26 |
| Magnesium stearate | 1,5 | 2,52 | 1,26 | 0,63 |
| Capsule | - | Gelatin | Gelatin | Gelatin |

### Example 3 Description of manufacturing process:

Granulate is manufactured in 3 parts (Part A, Part B and Part C), each part is prepared according to previous manufacturing steps (from materials weighing to screening of dry granulate).
Preparation of raw materials: sunitinib malate, mannitol, croscarmellose sodium, povidone K-25, hypromellose, magnesium stearate are weighted and screened using 0.5 - 1.0 screen.
Preliminary blending: Previously weighted sunitinib malate, mannitol, croscarmellose sodium, povidone K-25, hypromellose are placed into high shear granulator using impeller speed of about 150-300RPM and chopper speed of about 0-500 RPM and mixed for about 3 min.
Screening of the blend: The blend is calibrated through the screen 0.5 - 1.0 mm size.
Blending: Blend is mixed in high-shear mixer using impeller speed of about 250RPM and chopper speed of about 300 RPM within 1.5 min.
Granulation: Blend is granulated. During first 4 min of mixing water is continuously added using impeller speed of about 300-350RPM and chopper speed of about 0-800 RPM. Then during next 2.5 min wet granulate is mixed using impeller speed of about 325-375RPM and chopper speed of about 1000-2000 RPM to obtain homogenous mixture.
Wet granulate screening: Wet granulate is calibrated using 2.5 mm screen size.
Granulate drying: Granulate is dried in 60°C and mixed. Drying process is carried out until LOD (loss on drying) value is between 0.7 to 1.6% (IPC-1).
Dried granulate screening: Dried granulate is calibrated using 0.5-1.0 screen.
Granulate blending after addition of magnesium stearate: Earlier prepared parts A, B and C of granulate are combined in one mixture and next magnesium stearate is added.
Calculated portion of magnesium stearate is weighted and calibrated through the screen 0.5 mm in to the mixer with mixture of granulate (A, B and C). Mixture is blended in mixer for 2 min with 18RPM speed. The blend is calibrated through the screen 0.5-1.0 size and blended in mixer for 3 min. The final blend is analyzed for appearance.
Capsule filling: Hard capsules are filled with the appropriate mass of the blend:
- 168 mg of blend in case of 50 mg strength;
- 84 mg of blend in case of 25 mg strength;
- 42 mg of blend in case of 12.5 mg strength.
The compressed capsules are analyzed for appearance, average and single mass of capsule.

The similarity between the tested product and the reference product (Sutent®) was confirmed in the dissolution studies and additionally in the bioequivalence study.

Comparative dissolution profile testing was conducted for all three strengths composition according to the invention - Sunitinib product (50 mg, 25 mg, 12,5 mg) manufactured in - commercial scale batches Dissolution was investigated at different pH values (pH 1.2, 4.5 and 6.8).

According to Appendix I of the *"Guideline on the Investigation of Bioequivalence" (CPMP*/*EWP*/*QWP*/*1401198)*:
- "Where more than 85% of the drug is dissolved within 15 minutes, dissolution profiles may be accepted as similar without further mathematical evaluation;
- An *f₂* value between 50 and 100 suggests that the two dissolution profiles are similar". At 1.2 reference product and all three strengths of tested product dissolved more than 85% of the drug within 15 minutes.
At 4.5 and pH 6.8 *f₂* values are up to 50.
In this case due to the dissolution results of commercial batches of tested products, it could be concluded that the reference drug product and tested products could be assumed similar. 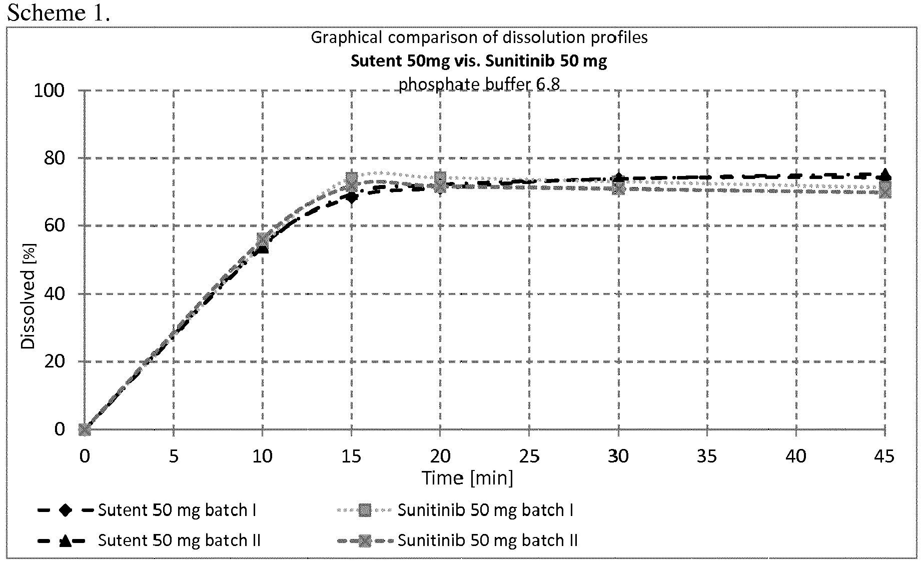 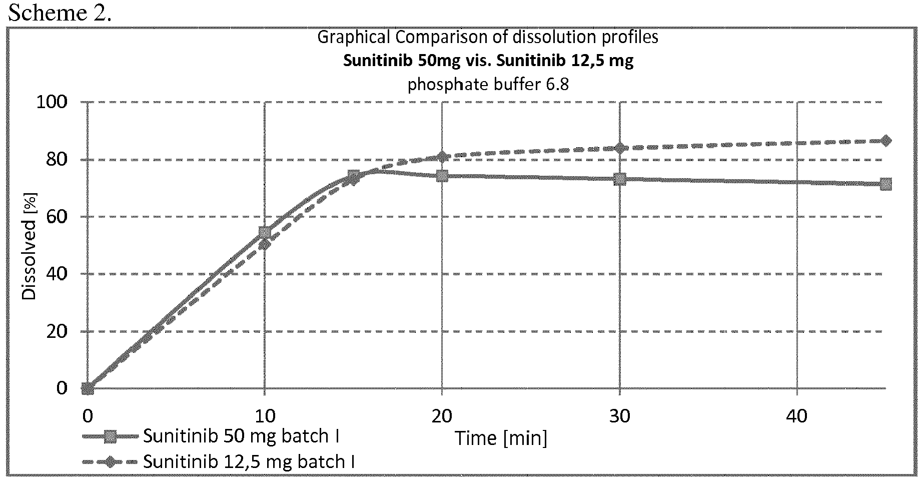 There was no similarity in dissolution profiles between medicinal product Sutent 12,5 mg and Sutent 50 mg.

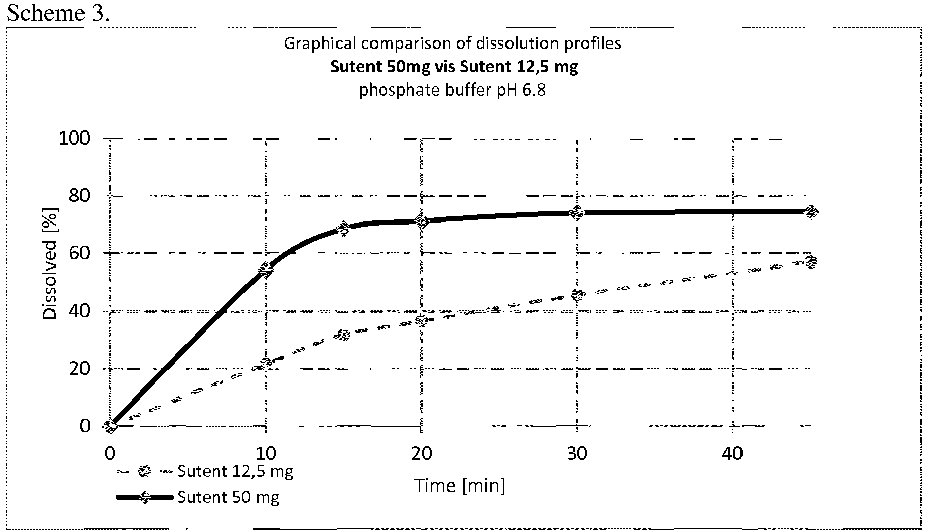

The similarity in dissolution studies of all strengths for tested product and lack of similarity in dissolution studies of all strengths for reference medicinal product might be more beneficial for tested product. Manufacturing of all three strengths from one common mixture of granulate is highly expected from economical point of view. Moreover, there is no need to conduct the bioequivalence studies for lower strengths which is beneficial from ethical reasons due to the exposure participants of clinical trial on high potent pharmaceutical active ingredient.

Detailed results of the dissolution studies are presented below.
For analysis of dissolution profiles validated HPLC method was used.
Dissolution studies were performed in three testing conditions:
- pH 1.2,
- pH 4.5,
- pH 6.8.
Percent dissolution values are mean values from 12 individual capsules.

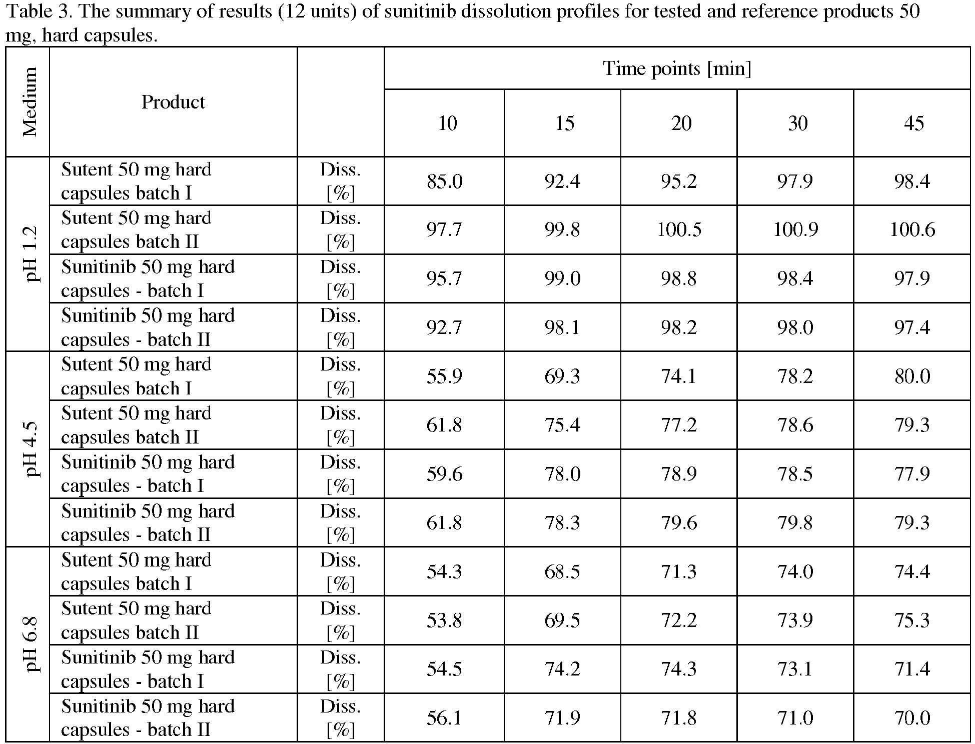

**Table 4. Comparison between tested and reference products (strength 50 mg), hard capsules by similarity factor (f2)**

| Product | | | pH 1.2 | pH 4.5 | pH 6.8 |
|---|---|---|---|---|---|
| | | | *f₂* | | |
| Sunitinib 50 mg hard capsules - batch I | vs | Sutent 50 mg hard capsules batch I | *NA | 63.5 | 73.4 |
| | | Sutent 50 mg hard capsules batch II | *NA | 83.4 | 77.8 |
| Sunitinib 50 mg hard capsules - batch II | vs | Sutent 50 mg hard capsules batch I | *NA | 60.4 | 78.7 |
| | | Sutent 50 mg hard capsules batch II | *NA | 82.4 | 80.8 |

| | | | | | |
|---|---|---|---|---|---|
| *NA - not applicable - more than 85% of the drug is dissolved within 15 minutes (Appendix I of the *"Guideline on the Investigation of Bioequivalence" CPMP*/*EWP*/*QWP*/*1401*/*98)* | | | | | |

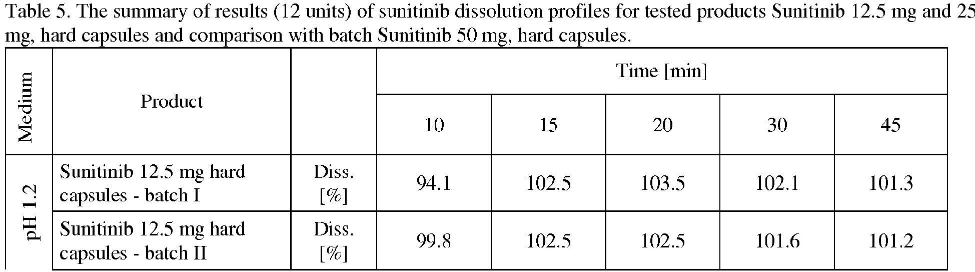

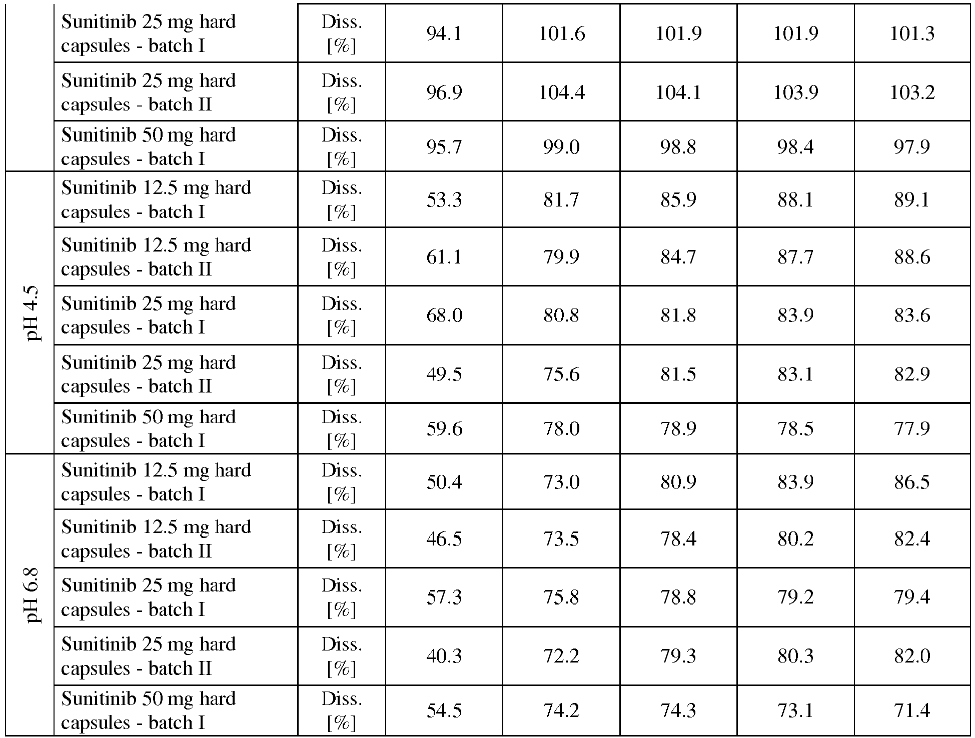

**Table 6. Comparison between tested product Sunitinib 50mg and lower strengths of Sunitinib by similarity factor (f2)**

| Product | | | pH 1.2 | pH 4.5 | pH 6.8 |
|---|---|---|---|---|---|
| | | | *f₂* | | |
| Sunitinib 12.5 mg hard capsules - batch I | vs | Sunitinib 50 mg hard capsules - batch I | *NA | 54.6 | 52.1 |
| Sunitinib 12.5 mg hard capsules - batch II | | | *NA | 57.8 | 57.2 |
| Sunitinib 25 mg hard capsules - batch I | | | *NA | 62.9 | 64.0 |
| Sunitinib 25 mg hard capsules - batch II | | | *NA | 62.1 | 52.4 |

According to Appendix I of the *"Guideline on the Investigation of Bioequivalence" (CPMP*/*EWP*/*QWP*/*1401*/*98)*:
- "Where more than 85% of the drug is dissolved within 15 minutes, dissolution profiles may be accepted as similar without further mathematical evaluation;
- An *f₂* value between 50 and 100 suggests that the two dissolution profiles are similar". At pH 1.2 reference product and all three strengths of tested product dissolved more than 85% of the drug within 15 minutes.
At pH 4.5 and pH 6.8 *f₂* values are up to 50.
In this case due to the dissolution results of - batches of tested products, it could be concluded that the reference drug product 50 mg and tested products in strengths 50mg, 25 mg, and 12,5 mg could be assumed similar in contrast to reference product which is not similar to all tested strenghts
Additionally, bioequivalence of the tested product comprising sunitinib maleate polymorph I 50mg with reference medicinal product - Sutent® 50 mg has been proven in clinical trials.
According to Guideline on the Investigation of Bioequivalence CPMP/EWP/QWP/1401/98 Rev. 1 /Corr** two medicinal products containing the same active substance are considered bioequivalent if they are pharmaceutically equivalent or pharmaceutical alternatives and their bioavailabilities (rate and extent) after administration in the same molar dose lie within acceptable predefined limits. These limits are set to ensure comparable *in vivo* performance, i.e. similarity in terms of safety and efficacy.
Pharmacokinetic parameters to be investigated during bioequivalence studies are as follows:
AUC - area under the plasma concentration curve
Cₘₐₓ - maximum plasma concentration
tₘₐₓ - time until cₘₐₓ is reached
In order to determine bioequivalence after a single dose, the parameters to be analysed are Cₘₐₓ, AUC₍₀₋ₜ₎, or when relevant, AUC₍₀₋₇₂ₕ₎. For these parameters, the 90% confidence interval for the ratio of the test and reference products values should be contained within the acceptance interval of 80.00-125.00%.
The results obtained in bioequivalence studies meet the required criteria for the above mentioned parameters. Therefore, the bioequivalence between two medicinal products was proved in terms of safety and efficacy.

The high quality of the pharmaceutical composition according to the invention was confirmed in stability studies conducted for all three strengths of tested product (50mg, 25mg, 12,5mg). The pharmaceutical composition according to the invention gives no significant change in storage stability when stored at 40°C±2°C and 75%±5% relative humidity for 6 months.
For analysis of impurities validated High Pressure Liquid Chromatography (HPLC) method was used.
The results for tested product after 6 months in accelerated conditions (40°C±2°C, 75%±5% RH) and after 24 months in long term conditions (25°C±2°C, 60%±5% RH (relative humidity) are presented below.
- Chemical max. single impurities in all three strengths and in all two stability conditions is presented in tables below:

**Table 7. Results of chemical purity study of Sunitinib 12.5 mg conducted in accelerated conditions.**

| | 12.5 mg_40°C±2°C, 75%±5% RH | | | |
|---|---|---|---|---|
| | batch I | | batch II | |
| Time [month] | Max. single impurity [%] | Total impurities [%] | Max. single impurity [%] | Total impurities [%] |
| Shelf-life limit | 0.2% | 1.5% | 0.2% | 1.5% |
| 0 | 0.14 | 0.37 | 0.14 | 0.37 |
| 1 | 0.16 | 0.39 | 0.14 | 0.38 |
| 3 | 0.15 | 0.39 | 0.15 | 0.40 |
| 6 | 0.15 | 0.48 | 0.16 | 0.47 |

**Table 8. Results of chemical purity study of Sunitinib 25 mg conducted in accelerated conditions.**

| | 25 mg_40°C±2°C, 75%±5% RH | | | |
|---|---|---|---|---|
| | batch I | | batch II | |
| Time [month] | Max. single impurity [%] | Total impurities [%] | Max. single impurity [%] | Total impurities [%] |
| Shelf-life limit | 0.2% | 1.5% | 0.2% | 1.5% |
| 0 | 0.14 | 0.37 | 0.14 | 0.38 |
| 1 | 0.15 | 0.39 | 0.14 | 0.38 |
| 3 | 0.15 | 0.40 | 0.15 | 0.44 |
| 6 | 0.16 | 0.46 | 0.16 | 0.45 |

**Table 9. Results of chemical purity study of Sunitinib 50 mg conducted in accelerated conditions.**

| | 50 mg_40°C±2°C, 75%±5% RH | | | |
|---|---|---|---|---|
| | batch I | | batch II | |
| Time [month] | Max. single impurity [%] | Total impurities [%] | Max. single impurity [%] | Total impurities [%] |
| Shelf-life limit | 0.2% | 1.5% | 0.2% | 1.5% |
| 0 | 0.15 | 0.34 | 0.15 | 0.31 |
| 1 | 0.16 | 0.40 | 0.15 | 0.34 |
| 3 | 0.17 | 0.35 | 0.15 | 0.39 |
| 6 | 0.16 | 0.4 | 0.16 | 0.40 |

**Table 10. Results of chemical purity study of Sunitinib 12.5 mg conducted in long term conditions.**

| | 12.5 mg_25°C±2°C, 60%±5% RH | | | |
|---|---|---|---|---|
| | batch I | | batch II | |
| Time [month] | Max. single impurity [%] | Total impurities [%] | Max. single impurity [%] | Total impurities [%] |
| Shelf-life limit | 0.2% | 1.5% | 0.2% | 1.5% |
| 0 | 0.14 | 0.37 | 0.14 | 0.37 |
| 3 | 0.15 | 0.38 | 0.15 | 0.39 |
| 6 | 0.17 | 0.39 | 0.16 | 0.40 |
| 9 | 0.15 | 0.48 | 0.15 | 0.53 |
| 12 | 0.16 | 0.45 | 0.15 | 0.45 |
| 18 | 0.16 | 0.40 | 0.16 | 0.41 |
| 24 | 0.15 | 0.46 | 0.15 | 0.46 |

**Table 11. Results of chemical purity study of Sunitinib 25 mg conducted in long term conditions.**

| | 25 mg_25°C±2°C, 60%±5% RH | | | |
|---|---|---|---|---|
| | batch I | | batch II | |
| Time [month] | Max. single impurity [%] | Total impurities [%] | Max. single impurity [%] | Total impurities [%] |
| Shelf-life limit | 0.2% | 1.5% | 0.2% | 1.5% |
| 0 | 0.14 | 0.37 | 0.14 | 0.38 |
| 3 | 0.16 | 0.33 | 0.15 | 0.38 |
| 6 | 0.15 | 0.34 | 0.15 | 0.38 |
| 9 | 0.15 | 0.47 | 0.15 | 0.46 |
| 12 | 0.15 | 0.53 | 0.14 | 0.53 |
| 18 | 0.16 | 0.57 | 0.16 | 0.56 |
| 24 | 0.15 | 0.61 | 0.15 | 0.59 |

**Table 12. Results of chemical purity study of Sunitinib 50 mg conducted in long term conditions.**

| | 50 mg_25°C±2°C, 60%±5% RH | | | |
|---|---|---|---|---|
| | batch I | | batch II | |
| Time [month] | Max. single impurity [%] | Total impurities [%] | Max. single impurity [%] | Total impurities [%] |
| Shelf-life limit | 0.2% | 1.5% | 0.2% | 1.5% |
| 0 | 0.15 | 0.34 | 0.15 | 0.31 |
| 3 | 0.15 | 0.38 | 0.15 | 0.34 |
| 6 | 0.16 | 0.40 | 0.16 | 0.39 |
| 9 | 0.15 | 0.48 | 0.14 | 0.52 |
| 12 | 0.16 | 0.47 | 0.17 | 0.53 |
| 18 | 0.17 | 0.53 | 0.16 | 0.51 |
| 24 | 0.16 | 0.50 | 0.16 | 0.50 |

After 6 months under accelerated conditions and after 24 months in long term condition:
- Assay of the active ingredient in capsules has not changed significantly (not exceeded 5% from its initial value).
- Dissolution of the active ingredient from capsules has not changed significantly.
- Requirements for other tests such as: appearance, average mass, have been also fulfilled.
- Microbial purity results in accelerated condition have been also fulfilled.
- Also, polymorphic form of the active ingredient in capsules is stable (by XRPD test).

## Claims

1. A pharmaceutical composition of sunitinib or its salt thereof in its polymorphic form I allowing for preparation of all proportional doses of sunitinib composition for administration to a patient comprising diluent, disintegrant, binder, lubricant wherein the composition comprises a macromolecular polymer - hypromellose (HPMC) in amount of 1-5% by weight of the total weight of the composition.

2. The pharmaceutical composition according to claim 1, wherein sunitinib is sunitinib maleate.

3. The pharmaceutical composition according to claim 1, hypromellose (HPMC) having viscosity (2% in water) in the range from 500 to 10000 mPa*s.

4. The pharmaceutical composition according to claim 1 or 2, comprising the following components:
- sunitinib malate 35-45 % w/w,
- mannitol 35-45% w/w,
- croscarmellose sodium 5-10% w/w,
- povidone (K-25) 2-8% w/w,
- hypromellose 1-5% w/w,
- magnesium stearate 1-2% w/w.

5. The pharmaceutical composition according to claim 3 comprising the following components:
- sunitinib malate 39,8 % w/w,
- mannitol 42,7 % w/w,
- croscarmellose sodium (8% intragranular), 8,0% w/w,
- povidone (K-25) 5,0% w/w,
- hypromellose 3,0% w/w,
- magnesium stearate 1,5% w/w.

6. The pharmaceutical composition according to any of the preceding claims, having bulk density in the range from 0.35 to 0.50 g/cm³.

7. The pharmaceutical composition according to any of the preceding claims, having Hausner ratio of 1.1 to 1.4.

8. The pharmaceutical composition according to any of the preceding claims having the active ingredient in the form of particles having a d90 value of less than 150 µm, d50 within the range of 10 to 60 µm, d10 within the range of 0 to 10 µm.

9. The pharmaceutical composition according to any of the preceding claims, having the form of capsules.

10. A method for manufacture of pharmaceutical composition of claim 1 comprising wet granulation of sunitinib or its salt thereof in its polymorphic form I and diluent, disintegrant, binder, macromolecular polymer, which is hypromellose (HPMC), then the granulate is dried and calibrated and a lubricant is added into granulate, further the granulate is formulated into pharmaceutical form of capsule.

11. The method according to claim 7, wherein granulate is dried in 40 - 70°C.
